# EUROPEAN PATENT APPLICATION

(11) **EP 4 317 172 A1**
(43) Date of publication of application: **07.02.2024**
(21) Application number: 22775658.2
(22) Date of filing: 23.03.2022
(51) Int. Cl.: C07K 7/06, C07K 16/18, G01N 33/53, G01N 33/574, C07K 14/78

(54) **IMMUNOLOGICAL ANALYSIS METHOD FOR TYPE-I COLLAGEN C-TERMINAL TELOPEPTIDE**

(30) Priority: 24.03.2021 JP 2021050231
(71) Applicant: Sekisui Medical Co., Ltd., Tokyo 103-0027 (JP)
(72) Inventor: HORIUCHI MORISHITA Shiomi, Tokyo 103-0027 (JP); OGASAWARA Rikako, Tokyo 103-0027 (JP); ASAI Tomohide, Tokyo 103-0027 (JP); MIYAZAKI Osamu, Tokyo 103-0027 (JP)
(74) Representative: Ter Meer Steinmeister & Partner
(86) International application number: PCT/JP2022/013387
(87) International publication number: WO 2022/202876

(57) **Abstract**

The problem addressed by the present invention is to provide an immunological assay method using a monoclonal antibody and a kit including the monoclonal antibody, which can be used for assaying ICTP without requiring special facilities. This problem can be solved by a method for immunological assay of type I collagen C-terminal telopeptide in a biological sample, including contacting type I collagen C-terminal telopeptide with a monoclonal antibody that recognizes an amino acid sequence represented by GFDFSFLP (SEQ ID NO: 1) as an epitope.

## Description

### [Technical Field]

The present invention relates to an immunological assay method for type I collagen C-terminal telopeptide. The present invention also relates to an assay kit for assaying type I collagen C-terminal telopeptide. The present invention also relates to a monoclonal antibody that recognizes a specific epitope in the type I collagen C-terminal telopeptide.

### [Background Art]

Type I collagen C-terminal telopeptide (hereinafter also referred to as ICTP) is a peptide produced during the degradation process of type I collagen, which is the main component of bone matrix. ICTP is produced through cleavage of the type I collagen by MMP (matrix metalloproteinase).

ICTP is one of bone resorption markers. The blood ICTP concentration is significantly higher in malignant tumors, particularly breast cancer, prostate cancer, or lung cancer cases with bone metastasis, than in cases without bone metastasis. This is considered to be because MMPs highly expressed in an environment with malignant tumors metastasized to bone promote decomposition of collagen molecules by osteoclasts, thereby promoting ICTP production. Therefore, the blood ICTP concentration is considered to be useful as a diagnostic aid or an indicator of therapeutic effects for bone metastasis of malignant tumors.

Pyridinoline ICTP (manufactured by Fujirebio Inc.) has been used as a reagent for measuring ICTP. However, this measuring reagent is employed in a radioimmunoassay using a polyclonal antibody. In consequence, special facilities for handling radioisotopes are required in order to use this measurement reagent. Therefore, development of a method and reagents that can be used for assaying ICTP without requiring special facilities has been desired.

Patent Literature 1 describes that an anti-ICTP polyclonal antibody is prepared using a sequence in ICTP as an antigen. This literature also describes that ICTP is analyzed using the anti-ICTP polyclonal antibody. However, a monoclonal antibody that can be used for measuring ICTP and an assay method using the monoclonal antibody are not disclosed. Monoclonal antibodies are generally more specific than polyclonal antibodies and have less cross-reactivity to substances structurally similar to the target substance to be detected.

### [Citation List]

### [Patent Literature]

Patent Literature 1: Japanese Unexamined Patent Application Publication (Translation of PCT Application) No. H8-509294

### [Summary of Invention]

### [Technical Problem]

The object of the present invention is to provide an immunological assay method using a monoclonal antibody and a kit including the monoclonal antibody, which can be used for assaying ICTP without requiring special facilities.

### [Solution to Problem]

The present inventors have made intensive studies to solve the above problems. As a result, the present inventors have found that the above problems can be solved by using a monoclonal antibody that recognizes an amino acid sequence represented by GFDFSFLP (SEQ ID NO: 1) as an epitope, and have completed the present invention.

Specifically, the present invention is as follows.
<1> A method for immunological assay of type I collagen C-terminal telopeptide in a biological sample, including:
   contacting type I collagen C-terminal telopeptide with a monoclonal antibody that recognizes an amino acid sequence represented by GFDFSFLP (SEQ ID NO: 1) as an epitope.
<2> The method according to <1>, wherein the monoclonal antibody does not recognize an amino acid sequence represented by FDFSFLP (SEQ ID NO: 2) as an epitope.
<3> The method according to <1> or <2>, wherein a labeling substance is directly or indirectly bound to the monoclonal antibody.
<4> The method according to <3>, wherein the labeling substance is a metal complex, an enzyme, an insoluble particle, or a metal colloid.
<5> The method according to <3> or <4>, which further includes a step of measuring a signal generated by the labeling substance.
<6> The method according to any one of <1> to <5>, wherein the biological sample is blood, plasma, or serum.
<7> The method according to any one of <1> to <6>, wherein the biological sample is a biological sample of a subject having breast cancer, prostate cancer, or lung cancer, which has metastasized to bone.
<8> The method according to any one of <1> to <7>, wherein the immunological assay is electrochemiluminescence immunoassay, latex immunoturbidimetry, immunochromatography, or ELISA.
<9> The method according to any one of <1> to <8>, wherein the biological sample contains the type I collagen C-terminal telopeptide at a concentration of 0.1 ng/mL to 1 µg/mL.
<10> The method according to any one of <5> to <9>, which further includes a step of comparing the signal with a threshold value.
<11> A kit for assaying type I collagen C-terminal telopeptide in a biological sample, including:
   a monoclonal antibody that recognizes an amino acid sequence represented by GFDFSFLP (SEQ ID NO: 1) as an epitope.
<12> The kit according to <9>, wherein the monoclonal antibody does not recognize an amino acid sequence represented by FDFSFLP (SEQ ID NO: 2) as an epitope.
<13> The kit according to < 11 > or < 12>, wherein the biological sample is blood, plasma or serum.
<14> The kit according to any one of <11> to <13>, wherein the biological sample is a biological sample of a subject having breast cancer, prostate cancer, or lung cancer, which has metastasized to bone.
<15> The kit according to any one of <11> to <14>, which is for use in electrochemiluminescence immunoassay, latex immunoturbidimetry, immunochromatography or ELISA.
<16> The kit according to any one of <11> to <15>, wherein the biological sample contains the type I collagen C-terminal telopeptide at a concentration of 0.1 ng/mL to 1 µg/mL.
<17> A monoclonal antibody that recognizes an amino acid sequence represented by GFDFSFLP (SEQ ID NO: 1) as an epitope.
<18> The monoclonal antibody according to <17>, which does not recognize an amino acid sequence represented by FDFSFLP (SEQ ID NO: 2) as an epitope.

### [Advantageous Effects of Invention]

The present invention can provide an immunological assay method using a monoclonal antibody and a kit including the monoclonal antibody, which can be used for assaying ICTP without requiring special facilities.

### [Brief Description of Drawings]

FIG. 1 is a diagram showing an amino acid sequence of a peptide used as an immunogen.
FIG. 2 is a schematic diagram showing amino acid sequences of peptides used for epitope analysis.
FIG. 3 is a diagram showing reactivity of the obtained monoclonal antibodies to a synthetic peptide (N-terminal side).
FIG. 4 is a diagram showing the reactivity of the obtained monoclonal antibodies to a synthetic peptide (C-terminal side).
Fig. 5 is a schematic diagram showing the relationship between the epitope sequences for the obtained monoclonal antibodies and the amino acid sequence of a peptide used as an immunogen.
Fig. 6 is a graph showing the test results for the reactivity between S25206 antibody and a biological sample (serum of a patient with cancer with bone metastasis) by competitive ELISA.
Fig. 7 is a graph showing the test results for the reactivity between S25207 antibody and a biological sample (serum of a patient with cancer with bone metastasis) by competitive ELISA.
FIG. 8 is a graph showing the evaluation results for the correlation between the immunological assay method using the S25207 antibody and a pyridinoline ICTP reagent as a prior commercial product.

### [Description of Embodiments]

1. Method for immunological assay of type I collagen C-terminal telopeptide in biological sample

### (Biological sample)

Representative examples of the "biological sample" in the present invention include solid tissues and body fluids derived from living bodies (living organisms), and body fluids are preferably used as the biological sample. The biological sample in the present invention is more preferably blood, serum, plasma, urine, saliva, sputum, lacrimal fluid, ear discharge, or prostatic fluid, and even more preferably blood, serum, or plasma. The blood, serum, or plasma may be blood, serum, or plasma of a subject with breast cancer, prostate cancer, or lung cancer. The blood, serum, or plasma may be blood, serum, or plasma of a subject with breast cancer, prostate cancer, or lung cancer, from which bone metastasis is occurring.

In the context of the present specification, the term "bone metastasis" means metastasis to bone from a cancer developed at any tissue other than bones.

Examples of subjects from which the biological sample is to be collected include humans or animals (e.g., monkeys, dogs, cats, mice, guinea pigs, rats, and hamsters), of which humans are preferable. The biological sample may be a biological sample per se taken from a subject, or may be a sample obtained by subjecting a collected biological sample to treatments such as dilution and concentration that are usually performed. The person who collects and prepares a biological sample from a subject used in the present invention may or may not be identical to the person who performs the immunological assay method of the present invention. Further, the biological sample used in the present invention may be one collected or prepared during implementation of the present invention, or one previously collected or prepared and stored.

### (Type I collagen C-terminal telopeptide)

Type I collagen C-terminal telopeptide (hereinafter also referred to as ICTP) is a peptide produced during the degradation process of type I collagen, which is the main component of bone matrix. Type I collagen is formed through combination of two α1 chains (type I) and one α2 chain (type I). The N-terminal or C-terminal telopeptides of the α1 or α2 chains are crosslinked with pyridinoline or deoxypyridinoline to form a collagen fiber structure. When type I collagen is degraded by bone resorption, the telopeptide moieties are released into the blood as fragments while remaining crosslinked. On the other hand, the pyridinoline cross-linked fragment including the C-terminal part is called "type I collagen C-terminal telopeptide".

ICTP is one of bone resorption markers. The blood ICTP concentration is significantly higher in malignant tumors, particularly breast cancer, prostate cancer, or lung cancer cases with bone metastasis, than in cases without bone metastasis. Therefore, the blood ICTP concentration is considered to be useful as a diagnostic aid or an indicator of therapeutic effect for bone metastasis of malignant tumor.

### (Monoclonal antibody)

In the context of the present specification, the term "monoclonal antibody" refers to an antibody or antibody molecule that is obtained from clones derived from a single antibody-producing cell. In other words, in the context of the present specification, the term "monoclonal antibody" encompasses fragments possessing the function of monoclonal antibody as long as the effects of the present invention can be achieved. For example, the fragment possessing the function of monoclonal antibody encompasses a functional fragment including the Fab portion of a monoclonal antibody obtained by enzymatic digestion of the monoclonal antibody, a functional fragment including the Fab portion of a monoclonal antibody produced by genetic recombination, a functional fragment including scFv produced by phage display method, and the like.

In the context of the present specification, the "monoclonal antibody" may be, for example, IgG, IgE, IgM, IgD, or IgA.

### (Amino acid sequence represented by SEQ ID NO: 1)

The monoclonal antibody used in the immunological assay method of the present invention is a monoclonal antibody that recognizes an amino acid sequence represented by GFDFSFLP (SEQ ID NO: 1) as an epitope (hereinafter also referred to as the monoclonal antibody of the present invention). The monoclonal antibody of the present invention is preferably a monoclonal antibody that specifically recognizes the amino acid sequence represented by GFDFSFLP (SEQ ID NO: 1). In this context, specifically recognizing the amino acid sequence represented by GFDFSFLP (SEQ ID NO: 1) means that the antibody does not substantially bind to or react with a peptide fragment having an amino acid sequence other than GFDFSFLP (SEQ ID NO: 1). In this case, the "peptide fragment having an amino acid sequence other than GFDFSFLP (SEQ ID NO: 1)" encompasses a peptide fragment whose amino acid sequence partially overlaps with GFDFSFLP (SEQ ID NO: 1) (e.g., a peptide fragment having an amino acid sequence represented by FDFSFLP (SEQ ID NO: 2)).

The monoclonal antibody of the present invention preferably does not recognize an amino acid sequence represented by FDFSFLP (SEQ ID NO: 2) as an epitope. Therefore, the monoclonal antibody of the present invention preferably does not substantially bind to a peptide fragment having the amino acid sequence represented by FDFSFLP (SEQ ID NO: 2).

The monoclonal antibody of the present invention more preferably does not recognize both of the amino acid sequence represented by FDFSFLP (SEQ ID NO: 2) and an amino acid sequence represented by SAGFDFSFL (SEQ ID NO: 3) as epitopes. Therefore, the monoclonal antibody of the present invention more preferably does not substantially bind to both of a peptide fragment having the amino acid sequence represented by FDFSFLP (SEQ ID NO: 2) and a peptide fragment having the amino acid sequence represented by SAGFDFSFL (SEQ ID NO: 3).

The monoclonal antibody of the present invention can be S25207 antibody or S25210 antibody. The monoclonal antibody of the present invention can be S25207 antibody.

The present inventors have found that the ICTP concentration in blood can be measured by using the monoclonal antibody of the present invention.

The inventors also have found that this monoclonal antibody recognizes, as an epitope, neither the amino acid sequence represented by FDFSFLP (SEQ ID NO: 2), which is a sequence excluding the first amino acid "G" from the N-terminal side of GFDFSFLP (SEQ ID NO: 1), nor the amino acid sequence represented by SAGFDFSFL (SEQ ID NO: 3), which does not have the terminal amino acid "P" and has "SAG" added on the N-terminal side of GFDFSFLP (SEQ ID NO: 1).

Furthermore, the present inventors have found that the ICTP concentration in blood cannot be measured by the use of a monoclonal antibody that recognizes as an epitope an amino acid sequence represented by SAGFDFS (SEQ ID NO: 4), which has an overlap with the amino acid sequence represented by GFDFSFLP (SEQ ID NO: 1).

It is surprising that a slight shift in amino acid sequence to be recognized significantly affects the blood ICTP concentration measuring performance by a monoclonal antibody. While not wishing to be bound by theory, it is possible that the three-dimensional structure and characteristic sequences of the amino acid sequence near SEQ ID NO: 1 affect recognition by monoclonal antibodies.

The present specification includes descriptions indicating that a monoclonal antibody "reacts with," "recognizes," or "binds to" a specific substance or amino acid sequence, which however are used interchangeably. Whether or not a monoclonal antibody "reacts with" an antigen (compound) can be determined through antigen-immobilized ELISA, competitive ELISA, sandwich ELISA, or the like. Alternatively, a method using the principle of surface plasmon resonance (SPR method) can also be used. The SPR method can be performed using equipment, sensors and reagents commercially available under the name Biacore (registered trademark).

For example, when the same operation as the competitive ELISA in the epitope analysis in the Examples described later is performed, a monoclonal antibody can be regarded as having recognized the amino acid sequence of the added peptide as an epitope when the signal is 70 % or less (preferably 60 % or less, more preferably 50 % or less) relative to the control to which no peptide is added.

### (Method for preparing monoclonal antibody)

The monoclonal antibody of the present invention can be prepared by dissolving, as an antigen (immunogen), a peptide fragment of ICTP including SEQ ID NO: 1, preferably a peptide fragment having a sequence represented by SAGFDFSFLFLPQPPQEKAHDGGRC (SEQ ID NO: 5), in a solvent such as phosphate-buffered saline, and administering the resulting solution to a non-human animal for immunization. A peptide fragment that is turned into a peptide fragment having the sequence represented by SEQ ID NO: 5 as a result of metabolization in the animal may be administered. Immunization may be performed using an emulsion after addition of an appropriate adjuvant to the solution as necessary. Examples of adjuvants include generally used adjuvants, such as water-in-oil emulsions, water-in-oil-in-water emulsions, oil-in-water emulsions, liposomes, and aluminum hydroxide gels, as well as proteins or peptide substances derived from biological components. For example, Freund's incomplete adjuvant or Freund's complete adjuvant can be suitably used. The administration route, dosage, and administration time for the adjuvant are not particularly limited, but are preferably selected appropriately so as to enhance the desired immune response in the animal to be immunized with the antigen.

The type of animal used for immunization is also not particularly limited, and mammals such as mice, rats, cows, rabbits, goats, sheep, or alpacas are preferred, and mice or rats are more preferred. Animals can be immunized following common techniques, for example, by subcutaneously, intracutaneously, intravenously, or intraperitoneally injecting an antigen solution, preferably a mixture thereof with an adjuvant, into the animals. Since the immune response generally differs depending on the type and strain of the animal to be immunized, it is desirable to appropriately set the immunization schedule according to the animal used. It is preferable to repeat the antigen administration several times after the initial immunization.

For obtaining the monoclonal antibody of the present invention, the following operations may be subsequently performed, which, however, are not essential and do not limit the present invention. Methods for producing monoclonal antibodies per se are well known and widely used in the art, and those skilled in the art can prepare the monoclonal antibody of the present invention by using the antigens as described above (see, for example, Antibodies, A Laboratory Manual (Cold Spring Harbor Laboratory Press, (1988), Chapter 6, etc.).

After the final immunization, antibody-producing spleen cells or lymph node cells are extracted from the immunized animal and fused with a myeloma-derived cell line having high proliferative potential to prepare hybridomas. Cells with high antibody-producing ability (in terms of quality and quantity) are preferably used for cell fusion, and it is more preferable that the myeloma-derived cell line is compatible with the animal from which the antibody-producing cells to be fused are derived. Cell fusion can be performed according to methods known in the art. For example, a polyethylene glycol method, a method using Sendai virus, a method using electric current, or the like can be employed. The resulting hybridomas can be grown according to conditions commonly employed in the art. A desired hybridoma can be selected while checking the properties of the antibody to be produced. Hybridoma cloning can be performed by well-known methods such as the limiting dilution method and the soft agar method.

After the cloning step, the ability of the produced monoclonal antibody to bind to the peptide fragment having the amino acid sequence represented by SEQ ID NO: 1 can be assayed by methods such as ELISA, RIA, and immunofluorescence. Absence of binding to the peptide fragment having the amino acid sequence represented by SEQ ID NO: 2 or 3 may be evaluated by a similar method. These operations allow determination of whether the selected hybridomas produce monoclonal antibodies with the desired properties.

By mass-culturing the hybridomas selected as described above, monoclonal antibodies having the desired properties can be produced. The method for mass-culturing is not particularly limited, and examples thereof include a method that cultures hybridomas in an appropriate medium to produce monoclonal antibodies in the medium, and a method that injects hybridomas into the peritoneal cavity of a mammal to allow the hybridomas to proliferate, thereby producing monoclonal antibodies in ascites.

As the monoclonal antibody of the present invention, it is possible to use a fragment of a monoclonal antibody having antigen-antibody reactivity as well as the whole antibody molecule. In addition to those obtained through the process of immunizing animals as described above, it is also possible to use monoclonal antibodies obtained using gene recombination techniques, such as chimeric antibodies and humanized antibodies. Fragments of monoclonal antibodies are preferably functional fragments, such as F(ab')2, Fab', scFv and the like. These fragments can be prepared by treating the monoclonal antibody obtained as described above with a protease (e.g., pepsin or papain), or cloning the DNA of the antibody and expressing it in a culture system using E. coli or yeast.

The immunological assay method of the present invention can also use a monoclonal antibody that recognizes an amino acid sequence in ICTP other than the amino acid sequence represented by GFDFSFLP (SEQ ID NO: 1) as an epitope (hereinafter also referred to as "monoclonal antibody that recognizes another amino acid sequence). Such a monoclonal antibody that recognizes another amino acid sequence can be easily prepared by a person skilled in the art by making necessary modifications to the aforementioned method for preparing a monoclonal antibody. Examples of necessary modifications include:
- immunizing with a peptide fragment having an amino acid sequence other than the amino acid sequence represented by GFDFSFLP (SEQ ID NO: 1) in ICTP, and
- assaying the ability to bind to a peptide fragment having a target amino acid sequence for binding.

When constructing the sandwich system described later, the monoclonal antibody that recognizes another amino acid sequence is preferably a monoclonal antibody that recognizes, as an epitope, an amino acid sequence that does not overlap with GFDFSFLP (SEQ ID NO: 1), considering the measurement principle of the sandwich system.

The monoclonal antibody that recognizes another amino acid sequence can also be used to construct a sandwich system in the immunological assay method. A sandwich system is a method that achieves high specificity and sensitivity by sandwiching a detection target substance between two types of antibodies that recognize different epitopes.

The sandwich system uses one antibody as a solid phase antibody and the other as a labeled antibody. In the context of the present specification, the "solid phase antibody" means a monoclonal antibody immobilized on a solid phase. In the context of the present specification, the "labeled antibody" means a monoclonal antibody that is directly or indirectly labeled with a conventional labeling substance well known in the art described below when measuring a signal attributable to the labeling substance.

When constructing a sandwich system, it is preferable that at least one of the two types of monoclonal antibodies is a solid-phase antibody, and at least one is preferably a labeled antibody.

For example, a solid-phase antibody can be produced by allowing a monoclonal antibody to physically adsorb or chemically bind to a solid phase (optionally via an appropriate spacer). The solid phase to be used may be a solid phase composed of a polymer substrate such as polystyrene resin, an inorganic substrate such as glass, a polysaccharide substrate such as cellulose or agarose, or the like. The shape of the solid phase is not particularly limited, and any appropriate shape may be chosen, for example, from a plate shape (e.g., microplate or membrane), a bead or particulate shape (e.g., latex particles, magnetic particles), or a cylindrical shape (e.g., test tube).

The use of a labeled antibody (secondary antibody) capable of directly binding to the monoclonal antibody used in the immunological assay method of the present invention also enables the measurement of amount of antibody bound to ICTP. In the present specification, an antibody bound to the monoclonal antibody of the present invention or the monoclonal antibody that recognizes another amino acid sequence and also bound to a labeling substance is referred to as a "secondary antibody". This secondary antibody may be used to indirectly bind the labeling substance to the monoclonal antibody used in the immunological assay method of the present invention.

By measuring the intensity of the signal generated by the labeling substance, the amount of ICTP in the biological sample can be measured. Examples of labeling substances for producing the labeled antibody include metal complexes, enzymes, insoluble particles, fluorescent substances, chemiluminescent substances, biotin, avidin, radioactive isotopes, colloidal gold particles, and colored latex. The method used for binding the labeling substance to the monoclonal antibody may be glutaraldehyde method, maleimide method, pyridyl disulfide method, or periodic acid method, which are available to those skilled in the art. The types of the solid-phase antibody and the labeled antibody, and the methods for producing them are not limited to the above examples. For example, when an enzyme such as horseradish peroxidase (HRP) or alkaline phosphatase (ALP) is used as a labeling substance, enzymatic activity can be measured using a specific substrate for the enzyme (for example, O-phenylenediamine (OPD) or 3,3',5,5'-tetramethylbenzidine (TMB) when the enzyme is HRP, and p-nitrophenyl phosphate when the enzyme is ALP). When biotin is used as a labeling substance, a monoclonal antibody may be labeled with biotin and reacted with avidin or streptavidin labeled with an enzyme, dye, or fluorescent label (preferably HRP). In the immunological assay method of the present invention, biotin or HRP is preferably used as the labeling substance.

In the context of the present specification, the process of physically or chemically supporting an antigen or antibody on a solid phase or the state of an antigen or antibody being supported on a solid phase is sometimes referred to as "immobilization" or " solid-phase immobilization". The term "assay", "detection" or "measurement" also encompasses proving the presence of ICTP and quantification of ICTP.

The immunological assay method of the present invention may use only a monoclonal antibody that binds to a single type of ICTP, that is, the monoclonal antibody of the present invention. Such assay method may be, for example, competitive ELISA including the following steps (1) to (4):
(1) immobilizing ICTP or a peptide fragment having the amino acid sequence represented by SEQ ID NO: 1 on a solid phase such as a microplate,
(2) adding a biological sample to be assayed to the microplate,
(3) adding an enzyme-labeled monoclonal antibody of the present invention to the microplate, and
(4) adding a substrate for the enzyme, and measuring a signal attributable to the enzymatic reaction.

When competition occurs between ICTP in the biological sample and ICTP immobilized on the solid phase, the intensity of the signal decreases.

Two types of monoclonal antibodies can also be used in the immunological assay method of the present invention. In this context, the "two types of monoclonal antibodies" means the monoclonal antibody of the present invention and a monoclonal antibody that recognizes another amino acid sequence in ICTP. When using the two types of monoclonal antibodies, the monoclonal antibody of the invention is referred to as "first monoclonal antibody". The monoclonal antibody that recognizes another amino acid sequence in ICTP is referred to as "second monoclonal antibody". Of the two types of monoclonal antibodies, at least one is a solid phase antibody and at least one is a labeled antibody. In this instance, the immunological assay method of the present invention may include the following steps (1) to (3):
(1) a step of contacting a biological sample with a first or second monoclonal antibody to form a first complex,
(2) a step of contacting the first complex with a first or second monoclonal antibody different from that used in step (1) to form a second complex, and
(3) a step of measuring a signal attributable to the labeling substance.

The second complex contains a labeling substance. For signal measurement, a measurement method well known in the art can be employed depending on the labeling substance.

When using one or two types of monoclonal antibodies, the immunological assay method of the present invention may include, if necessary, a step of pretreating the biological sample and/or a step of comparing the strength of the obtained signal with a first threshold. The first threshold may be appropriately set in consideration of sensitivity and specificity.

The first threshold may be a numerical range. The first threshold being a numerical range means that the specified range include a specific threshold, and the determination of whether the measured value is larger or smaller than the specific threshold allows determination of the presence or absence of a disease. For example, when the first threshold ranges from 3 ng/mL to 6 ng/mL, and the specific threshold is 4 ng/mL to 6 ng/mL, the specific threshold may be 4.5 ng/mL, 5 ng /mL, 5.5 ng/mL, and so forth.

Alternatively, as in the case of "range threshold" described later, the presence or absence of a disease may be determined by determining whether or not the measured value falls within or outside a numerical range such as 4 ng/mL to 6 ng/mL.

The lower limit of the first threshold may be, for example, 0.1 ng/mL, 0.5 ng/mL, 1 ng/mL, 2 ng/mL, 3 ng/mL, 4 ng/mL, 4.5 ng/mL, 5 ng/mL, 6 ng/mL, 7 ng/mL, 8 ng/mL, 9 ng/mL, 10 ng/mL, 25 ng/mL, 50 ng/mL, or 100 ng/mL. The upper limit of the first threshold may be 100 ng/mL or less, 50 ng/mL or less, 25 ng/mL, 10 ng/mL, 9 ng/mL, 8 ng/mL, 7 ng/mL, 6 ng/mL, 5 ng/mL, 4.5 ng/mL, 4 ng/mL, 3 ng/mL, 2 ng/mL, 1 ng/mL, or 0.5 ng/mL. Examples of specific first threshold ranges include a range of 0.1 ng/mL to 100 ng/mL, a range of 0.5 ng/mL to 50 ng/mL, a range of 1 ng/mL to 10 ng/mL, a range of 3 ng/mL to 6 ng/mL, and a range of 4 ng/mL to 5 ng/mL.

The first threshold may be a specific numerical value. Examples of specific numerical values include 4 ng/mL, 4.1 ng/mL, 4.2 ng/mL, 4.3 ng/mL, 4.4 ng/mL, 4.5 ng/mL, 4.6 ng/mL, 4.7 ng /mL, 4.8 ng/mL, 4.9/mL, 5 ng/mL, 5.1 ng/mL, 5.2 ng/mL, 5.3 ng/mL, 5.4 ng/mL, 5.5 ng/mL, 5.6 ng/mL, 5.7 ng/mL, 5.8 ng/mL, and 5.9 ng/mL.

The immunological assay method of the present invention may include a step of determining that breast cancer, prostate cancer, or lung cancer has metastasized to bone, when the signal strength is lower (higher) than the first threshold, or a step of determining that breast cancer, prostate cancer, or lung cancer has not metastasized to bone, when the signal strength is higher (lower) than the first threshold.

The immunological assay method of the present invention can determine the therapeutic effect of a specific drug (e.g., anti-tumor agent) on breast cancer, prostate cancer, or lung cancer patients based on the measured signal values. In this instance, the immunological assay method of the present invention may further include the following step(s) in addition to the steps described above:
a step of administering a specific drug (e.g., antitumor agent) to the subject, and/or
a step of comparing the strength of the signal to a second threshold.

In this instance, the second threshold may be set as appropriate, taking into account sensitivity and specificity, but may be the measured ICTP in the subject prior to administration of a specific drug (e.g., an antitumour agent) to the subject.

The immunological assay method of the present invention may include a step of determining that a specific drug (e.g., antitumor agent, etc.) has a therapeutic effect when the signal strength is lower (higher) than the second threshold, or a step of determining that a specific drug (e.g., antitumor agent, etc.) has no therapeutic effect when the signal strength is higher (lower) than the second threshold.

Examples of the antitumor agents include bisphosphonates, anti-RANKL antibodies (denosumab), RANKL antagonists (osteoprotegerin), and TGF-β kinase inhibitors (galunisertib).

In the determination of the therapeutic effect, the therapeutic effect may be monitored by conducting measurement every few days or weeks.

The first threshold and the second threshold may each take the form of a "range threshold". The "range threshold" means that the presence or absence of a disease is determined based on whether or not the measured value falls within the range, rather than whether the measured value is larger or smaller than a specific numerical value.

### (Immunological assay method)

Examples of the immunological assay method of the present invention include, but not limited to, electrochemiluminescence immunoassay (ECL method), enzyme-linked immunosorbent assay (ELISA), latex immunoturbidimetric assay (LTIA method), chemiluminescence immunoassay, immunochromatography, and immunofluorescence. The immunological assay method of the present invention is preferably electrochemiluminescence immunoassay, ELISA, latex immunoturbidimetry, or immunochromatography.

The immunological assay method of the present invention can be an in vivo or in vitro immunological assay method. Further, a sensitizer may also be used to enhance sensitivity.

The immunological assay method of the present invention can assay ICTP contained in a biological sample at a concentration of 0.1 ng/mL to 1 µg/mL, 0.2 ng/mL to 500 ng/mL, 0.3 ng/mL to 200 ng/mL, 0.5 ng/mL to 150 ng/mL, 1 ng/ml to 120 ng/ml, or 1 ng/ml to 100 ng/ml.

In the immunological assay method of the present invention, the order of adding the monoclonal antibody of the present invention and the biological sample or type I collagen C-terminal telopeptide to the measurement system is not limited as long as the effects of the present invention can be achieved. That is, the monoclonal antibody of the present invention may be added to the measurement system before the addition of the biological sample or the type I collagen C-terminal telopeptide, or simultaneously with the addition of the biological sample or the type I collagen C-terminal telopeptide, or after the addition of the biological sample or the type I collagen C-terminal telopeptide.

The measurement procedure and principle are described below for each immunological assay method to be employed. The following descriptions are presented for illustrative purpose only with respect to the measurement procedure and principle for one embodiment of the present invention, and by no means limit the scope of the present invention.

In each of the immunological assay methods described below, methods known in the art including those described above can be used without any limitation with respect to specific methods such as the method for immobilizing the monoclonal antibody on the solid phase, the method for binding the monoclonal antibody to the labeling substance, and the type of labeling substance.

In the example below, the first monoclonal antibody is used as the solid-phase antibody and the second monoclonal antibody is used as the labeled antibody, but these are interchangeable as long as the effects of the present invention can be achieved. That is, the first monoclonal antibody may be used as the labeled antibody and the second monoclonal antibody may be used as the solid-phase antibody.

### (Electrochemiluminescence immunoassay)

Electrochemiluminescence immunoassay means a method in which a labeling substance is caused to emit light by application of electric current, and the amount of light emitted is detected to measure the amount of a target substance to be detected. A ruthenium complex can be used as a labeling substance in the electrochemiluminescence immunoassay. An electrode is placed on a solid phase (such as a microplate), and radicals are generated on the electrode to excite the ruthenium complex to emit light. Then, the amount of light emitted from this ruthenium complex can be detected.

The measurement procedure and principle when using the first monoclonal antibody as the solid phase antibody, the second monoclonal antibody as the labeled antibody, the magnetic particles as the solid phase, and the ruthenium complex as the labeling substance are as follows.
(1) When the magnetic particles on which the solid-phase antibody is immobilized are brought into contact with the biological sample, ICTP in the biological sample binds to the solid-phase antibody.
(2) When the labeled antibody is brought into contact with the magnetic particles after washing, the labeled antibody binds to the ICTP bound to the magnetic particles.
(3) After the magnetic particles are washed, the application of electric current causes light emission depending on the amount of the labeled antibody bound to ICTP. By measuring the amount of luminescence, the amount of the target substance to be detected in the biological sample can be accurately measured.

### (ELISA)

Among various immunological assay methods, ELISA using an enzyme label is also preferable because the target can be measured easily and quickly. In the context of the present specification, the "ELISA" means a method in which an antigen or antibody as a target substance to be detected contained in a sample is trapped with an antibody or antigen corresponding to the target substance, and then detected using an enzymatic reaction. For the sandwich ELISA, the first monoclonal antibody may be used as the solid-phase antibody, or the second monoclonal antibody may be used as the solid-phase antibody. The solid phase is preferably a plate (immunoplate). HRP or ALP can be used as a label. The measurement procedure and principle when the sandwich ELISA is used as the immunological assay method of the present invention are as follows.
(1) When a biological sample is added to a solid phase on which a solid-phase antibody is immobilized and allowed to react, ICTP in the biological sample binds to the solid-phase antibody to form a solid-phase antibody-ICTP complex on the solid phase.
(2) When a labeled antibody that recognizes another labeled epitope is added to the solid phase and allowed to react, the labeled antibody binds to the trapped ICTP and forms a sandwich with the above solid-phase antibody-ICTP complex.
(3) After washing, the resulting is reacted with an enzyme substrate to develop color, and the absorbance is measured.

The amount of ICTP in the biological sample can be measured according to the amount of labeling substance measured.

A secondary antibody can also be used in the sandwich ELISA. The use of a secondary antibody can amplify the reaction and enhance the detection sensitivity. When using a secondary antibody, the following procedures (1) to (4) can be adopted:
(1) a first monoclonal antibody as a primary antibody,
(2) a solid phase on which a second monoclonal antibody is immobilized,
(3) an antibody (secondary antibody) corresponding to the first monoclonal antibody, labeled with an enzyme (such as HRP or ALP), and
(4) an enzyme substrate (such as OPD, TMB, or p-nitrophenyl phosphate, etc.).

First, a biological sample that has been appropriately treated and diluted is added to a solid phase with a second monoclonal antibody immobilized thereon, followed by incubation, removal of the biological sample and washing. Subsequently, a primary antibody is added, incubated and washed, and an enzyme-labeled secondary antibody is added thereto and incubated. Then, a substrate is added for color development. Thereafter, the amount of ICTP can be measured by measuring color development using a plate reader or the like.

A competitive ELISA, which is a competitive method, can also be employed. A competitive method is a method in which a target substance to be detected in the biological sample is allowed to compete with a competitive substance immobilized on a solid phase, to thereby assay the target substance in the biological sample. Specifically, such a method can employ the following procedures (1) to (4):
(1) immobilizing ICTP or a peptide fragment having the amino acid sequence shown in SEQ ID NO: 1 on a solid phase such as a microplate;
(2) adding a biological sample to be assayed to the microplate,
(3) adding an enzyme-labeled monoclonal antibody of the present invention to the microplate, and
(4) adding a substrate for the enzyme, and measuring a signal attributable to the enzymatic reaction.

Biotin can also be used. Streptavidin labeled with HRP or the like may be bound to this biotin. Then, a chromogenic signal generated by addition of OPD as a substrate can be measured.

### (Latex immunoturbidimetry)

Latex immunoturbidimetry is an immunological assay method that utilizes agglutination of an antibody bound to the surface of latex and a target substance to be detected (antigen). The latex particles are not particularly limited as long as they are latex particles generally used for in vitro diagnostic agents. With respect to the latex particles during the agglutination reaction measurement, the concentration, average particle size and the like can be appropriately set according to the sensitivity or performance. The measurement procedure and principle when the latex immunoturbidimetry is used as the immunological assay method of the present invention are as follows.
(1) A first monoclonal antibody and a second monoclonal antibody are bound to latex particles and brought into contact with a biological sample.
(2) ICTP in the biological sample binds to the first monoclonal antibody and the second monoclonal antibody, resulting in agglutination of the antibody-bound latex particles.
(3) The biological sample is irradiated with near-infrared light to measure absorbance or scattered light. Based on the measurements, the antigen concentration can be determined.

When the latex immunoturbidimetry is used as the immunological assay method of the present invention, the latex is a solid phase and acts as a labeling substance as well. That is, both the first monoclonal antibody and the second monoclonal antibody are bound to each of the solid phase and the labeling substance.

### (Immunochromatography)

Immunochromatography is an immunological assay method that utilizes the behavior that a labeled antibody bound to a target substance to be detected or a labeled antibody flows on a membrane. A general principle in measuring a target substance to be detected by the immunochromatography is as follows.

An antibody against an antigen (target substance to be detected) is immobilized on an insoluble membrane carrier, which is a chromatographic medium, to prepare a detector unit, which is a stationary phase. Then, a labeling substance sensitized by an antibody, which is capable of binding to the target substance to be detected and is a conjugate (detection reagent), is used as a mobile phase. The target substance to be detected and the conjugate as a mobile phase are allowed to react specifically, and the resulting the target substance bound to the conjugate is allowed to react, in the detector unit as a stationary phase, specifically with the antibody immobilized on the detector unit.

The measurement procedure and principle when the immunochromatography is employed as the immunological assay method of the present invention are as follows.
(1) A biological sample is brought into contact with a sample supply unit for supplying the biological sample.
(2) ICTP in the biological sample and a conjugate are brought into contact to form a first complex. The conjugate is a labeling substance having a first or second monoclonal antibody attached thereto.
(3) The first complex is brought into contact with the second or first monoclonal antibody immobilized on the detector unit to form a second complex.
(4) The intensity of the signal attributable to the labeling substance contained in the conjugate is measured to confirm the formation of the second complex.

Examples of labeling substances include colloidal gold particles, colloidal platinum particles, color latex particles, magnetic particles, and the like. Those skilled in the art can appropriately select and adjust the type and particle size of these labeling substances according to the desired sensitivity.

### 2. Kit for assaying ICTP in biological sample

The kit of the present invention for assaying ICTP in a biological sample (hereinafter, also referred to simply as "assay kit of the present invention") includes the monoclonal antibody of the present invention.

The ICTP assay kit of the present invention may be an assay kit including only the monoclonal antibody of the present invention for use in a competitive method, preferably competitive ELISA.

Two types of monoclonal antibodies may also be used in the assay kit of the present invention. The two types of monoclonal antibodies are the monoclonal antibody of the present invention and a monoclonal antibody that recognizes another amino acid sequence. One of the above antibodies may be a labeled antibody, while the other may be a solid-phase antibody. In this instance, the two types of monoclonal antibodies may be placed in separate containers.

Examples of the assay kit of the present invention include, but not limited to, assay kits for performing electrochemiluminescence immunoassay, ELISA, latex immunoturbidimetry, chemiluminescence immunoassay, immunochromatography, and immunofluorescence. The assay kit of the present invention is preferably an assay kit for performing electrochemiluminescence immunoassay, ELISA, latex immunoturbidimetry or immunochromatography.

The assay kit of the present invention can be an assay kit for assaying in vivo or in vitro samples.

The assay kit of the present invention can also include other test reagents such as standard antigen substances and quality control antigen samples, specimen diluents, and/or an instruction manual, etc. A person skilled in the art can appropriately adjust the concentrations of the antibody-containing reagent and the like.

The reagents contained in the kit are described below for each immunological assay method to be employed. In the example below, the first monoclonal antibody is used as the solid-phase antibody and the second monoclonal antibody is used as the labeled antibody, but these are interchangeable as long as the effects of the present invention can be achieved.

When using the electrochemiluminescence immunoassay, the assay kit of the present invention may include the following (A) and (B):
(A) a labeling reagent including a conjugate of a second monoclonal antibody and an electrochemiluminescent substance (e.g., a ruthenium complex, etc.); and
(B) a solid phase having immobilized thereon a first monoclonal antibody that binds to ICTP.

For example, in a kit using magnetic particles as the solid phase, the biological sample is added to the magnetic particles on which the first monoclonal antibody that binds to ICTP is immobilized and allowed to react, followed by removing the biological sample and washing. The conjugate is then added and allowed to react. After washing the magnetic particles, electrical energy is applied to cause light emission. ICTP can then be assayed by measuring the amount of light emitted from the labeling substance.

When using sandwich ELISA, the assay kit of the present invention may include the following (A) and (B):
(A) a labeling reagent including a conjugate of a second monoclonal antibody and an enzyme (HRP, ALP, etc.); and
(B) a solid phase having a first monoclonal antibody immobilized thereon.

In such a kit, first, a biological sample is added to a solid phase with a first monoclonal antibody immobilized thereon, followed by incubation, removal of the biological sample, and washing. Next, a labeling reagent is added, followed by incubation and addition of a substrate for color development. ICTP can be assayed by measuring color development using a plate reader or the like.

A secondary antibody can also be used in sandwich ELISA. The use of a secondary antibody can amplify the reaction and enhance the detection sensitivity. When using a secondary antibody, the analysis kit of the present invention may include the following (A) to (D):
(A) a second monoclonal antibody as a primary antibody,
(B) a solid phase on which a first monoclonal antibody is immobilized,
(C) an antibody (secondary antibody) corresponding to the second monoclonal antibody, labeled with an enzyme (HRP or ALP, etc.), and
(D) an enzyme substrate (OPD, TMB, p-nitrophenyl phosphate, etc.).

In such a kit, first, a biological sample that has been appropriately treated and diluted is added to the solid phase on which the first monoclonal antibody is immobilized, followed by incubation, removal of the biological sample, and washing. Subsequently, the primary antibody is added, followed by incubation and washing. Further, an enzyme-labeled secondary antibody is added and incubated. Then, a substrate is added for color development. ICTP can be assayed by measuring color development using a plate reader or the like.

The solid phase and the first monoclonal antibody may be included separately in the assay kit. In this instance, the first monoclonal antibody is immobilized on the solid phase by an assay performer.

For competitive ELISA, the assay kit of the present invention may include the following (A) and (B):
(A) ICTP or a solid phase having immobilized thereon a peptide fragment having the amino acid sequence represented by SEQ ID NO: 1, and
(B) an enzyme-labeled monoclonal antibody of the present invention.

The solid phase and ICTP or the peptide fragment having the amino acid sequence represented by SEQ ID NO: 1 may be included separately in the assay kit. In this instance, ICTP or the peptide fragment having the amino acid sequence represented by SEQ ID NO: 1 is immobilized on the solid phase by an assay performer.

Biotin can also be used. Biotin may be bound to streptavidin leveled with HRP, etc. OPD may further be included as a substrate.

When using latex immunoturbidimetry, the assay kit of the present invention may include latex particles bound with a first monoclonal antibody and latex particles bound with a second monoclonal antibody. When the assay kit of the present invention is a kit for latex immunoturbidimetry, the latex serves as a solid phase and a labeling substance as well. Thus, both the first monoclonal antibody and the second monoclonal antibody are bound to each of the solid phase and the labeling substance.

When using immunochromatography, the assay kit of the present invention may take a form in which an immunochromatography test strip is stored and mounted in a suitable container (housing). The immunochromatography test strip may be composed of a sample pad having a sample supply unit, an insoluble membrane carrier as a chromatography medium, and an absorbent pad disposed at the downstream end of the insoluble membrane carrier. A detection unit with a first monoclonal antibody immobilized thereon may be placed on the insoluble membrane carrier, and a conjugate pad with a conjugate placed thereon may be placed between the sample pad and the insoluble membrane carrier. The conjugate may be placed on the sample pad or the insoluble membrane carrier. As regards other configurations of immunochromatography, for example, those described in International Publication No. 2018/012517 or International Publication No. 2016/031892 may be appropriately adopted.

Examples of labeling substances include colloidal gold particles, colloidal platinum particles, color latex particles, magnetic particles, and the like. Those skilled in the art can appropriately adjust the types and particle sizes of these labeling substances.

Thus, the descriptions are given above separately for various aspects of the invention, but the descriptions, definitions of terms, and embodiments described for a particular aspect are also applicable to the other aspects.

Hereinbelow, the present invention will be described in detail with reference to examples, which however should not be construed as limiting the present invention. In the following description, the unit "%" refers to "% by mass", unless otherwise specified.

### [Examples]

### <<Example 1 Preparation of monoclonal antibody>>

### 1-1 Used reagents, etc.

- Immunogens and antigens
- Synthetic peptide (IC01: SAGFDFSFLPQPPQEKAHDGGRC (SEQ ID NO: 5)): Protein Purify Co.
- Synthetic peptide (IC08: SAGFDFSFLPQPPQC (SEQ ID NO: 6)): Protein Purify Co.
- Synthetic peptide (IC09: CSAGFDFSFLPQPPQ (SEQ ID NO: 7)): Protein Purify Co.
- SM(PEG) 2,100 mg: Thermo, 22102
- Albumin from bovine serum: Sigma, A7906-10G
- Transferrin, Human, recombinant: Wako, 201-1808
- Slide-A-Lyzer Dialysis Cassette (MWCO: 10k): Thermo, 66380

### - SDS-PAGE

Protein transcription kit: Cosmo Bio Co., Ltd., 423536
- Sample Treatment for Tris SDS: Cosmo Bio, 423420
- Protein Multi Color Stable: Bio Dynamics Laboratory, DM660
- Multi Gel II mini 4/20 (13W): Cosmo Bio, 414879
- TaKaRa CBB Protein Safe Stain (G250): Takara Bio, T9320A
- Blocking solution, washing solution, substrate dissolution liquid, other reagents: used following existing prescriptions

### - Various ELISA

- Microplate for 96-well ELISA: NUNC 442404
- Goat anti-Mouse IgG (H+L) PAb-HRP: Southern Biotech, 1031-05, Lot F0415-NB76H
- Goat anti-Rat IgG (H+L) PAb-HRP: Southern Biotech, 3050-05, Lot G8212-PK14L
- Anti-Human CTXI Mouse MAb: Cloud Clone, MAA665Hu21
- Washing solution, blocking reagent, OPD coloring solution, stop solution, etc.: used following existing prescriptions

### - Specimen

- Cancer bone metastasis patient serum, value-assigned by pyridinoline ICTP (Fujirebio (Orion Diagnostica)), was used.

### - ICTP measurement reagent

- Pyridinoline ICTP: Fujirebio (Orion Diagnostica)

### 1-2 Preparation of immunogen

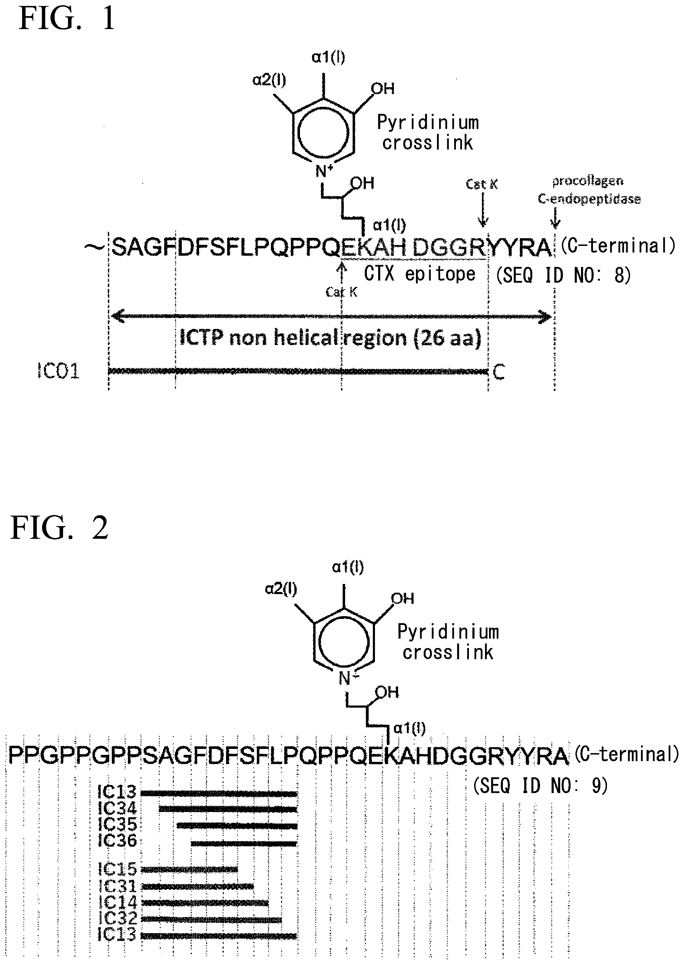

The amino acid sequence of the immunogenic peptide used is shown in FIG 1. An SM(PEG)2 linker was used for cross-linking the synthetic peptide and the carrier protein. A 10 mg/mL carrier protein (BSA, HTF) solution was prepared using PBS as a solvent. 60 equivalents or more of the SM(PEG)2 linker was added and the resulting was stirred with a rotator for 30 minutes at room temperature. After the reaction, the resulting solution was dialyzed twice against 500 mL/sample of PBS (4°C, 4h for the 1st round, overnight for the 2nd round). 60 equivalents of peptide as carrier-linker were dissolved in 100 µL of PBS. To 100 µL of the carrier-linker solution, an equal volume of peptide solution was added. The resulting was stirred with a rotator for 30 minutes at room temperature. After the reaction, the resulting solution was dialyzed twice against 500 mL/sample of PBS (4°C, 4h for the 1st round, overnight for the 2nd round).

### 1-3 Production of antibody

The peptide conjugate IC01-(PEG)2-HTF was used as immunogen. The immunogen was mixed at 1:1 with Freund's Complete Adjuvant (Difco Laboratories) for the first immunization and with Freund's Incomplete Adjuvant (Difco Laboratories) for the second and subsequent immunizations. For Balb/c, subcutaneous immunization was continuously carried out every other week using an immunogen amount of 50 µg for the initial round immunization, 20 µg for the second and subsequent rounds of immunization, and 50 µg (diluted with PBS) for the final round of immunization. The blood antibody titers were evaluated by antigen-immobilized ELISA after implementing the third round of immunization. Individuals showing a sufficient increase in titer were intraperitoneally immunized with an immunogen diluted with PBS one to three days before dissection. Then, spleen cells, iliac lymph node cells and inguinal lymph node cells were collected and fused with myeloma cell SP2/0 by electrofusion method. The fused cells were cultured on a 96-well plate. Seven or eight days after the fusion, the culture supernatant was collected and screened by antigen-immobilized ELISA described below. A strain was selected that showed reactivity to IC01-(PEG)2-BSA and no reactivity to Cys-(PEG)2-BSA. In this process, the medium was exchanged on the day before the screening.

### 1-4 Primary screening (antigen-immobilized ELISA)

(1) IC01-(PEG)2-BSA and Cys-(PEG)2-BSA (1 µg/mL in PBS) were dispensed (50 µL/well) into a 96-well plate for ELISA and allowed to stand at room temperature for 2 hours.
(2) After washing 3 times (400 µL/well), a blocking solution was dispensed (100 µL/well) and the resulting was allowed to stand at room temperature for 1 hour or at 4 °C overnight.
(3) After removing the blocking solution, a cell culture supernatant (2-fold diluted) and an antiserum (1000- and 10000-fold diluted) were dispensed (50 µL/well) and the resulting was allowed to stand at room temperature for 1 hour.
(4) After washing three times, Goat anti-Mouse IgG (H+L) PAb-HRP (9500-fold diluted) was dispensed (50 µL/well) and the resulting was allowed to stand at room temperature for 1 hour.
(5) After washing 3 times, an OPD coloring solution was dispensed (50 µL/well) and the resulting was allowed to stand at room temperature for 10 minutes.
(6) A stop solution was dispensed (50 µL/well), and after stopping the reaction, measurement was performed using a plate reader (Abs. 492 nm).
(7) As a result, S25206 antibody, S25207 antibody, S25209 antibody, and S25210 antibody were selected.

### 1-5 Epitope analysis

Epitope analysis of the acquired antibodies was performed by competitive ELISA using the peptides shown in FIG. 2.
(1) IC01-(PEG)2-BSA (10 ng/mL in PBS) was dispensed into a 96-well plate for ELISA (50 µL/well) and allowed to stand at room temperature for 2 hours.
(2) After washing 3 times (400 µL/well), a blocking solution was dispensed (100 µL/well) and the resulting was allowed to stand at room temperature for 1 hour or at 4 °C overnight.
(3) After removing the blocking solution, the peptides diluted with PBS (S25207 antibody: 1000 ng/mL, S25210 antibody: 200 ng/mL, S25206 antibody: 2 ng/mL, S25209 antibody: 313 ng/mL) were dispensed (25 µL/well), followed by further dispensing a purified antibodies prepared in-house (S25207 antibody: 50 ng/mL, S25210 antibody: 10 ng/mL, S25206 antibody: 9 ng/mL, S25209 antibody: 14 ng/mL) (25 µL/well), and the resulting was allowed to stand at room temperature for 1 hour.
(4) After washing three times, Goat anti-Mouse IgG (H+L) PAb-HRP (9500-fold diluted) was dispensed (50 µL/well) and the resulting was allowed to stand at room temperature for 1 hour.
(5) After washing 3 times, an OPD coloring solution was dispensed (50 µL/well) and the resulting was allowed to stand at room temperature for 10 minutes.
(6) A stop solution was dispensed (50 µL/well), and after stopping the reaction, measurement was performed using a plate reader (Abs. 492 nm).

### 1-6 Results of epitope analysis

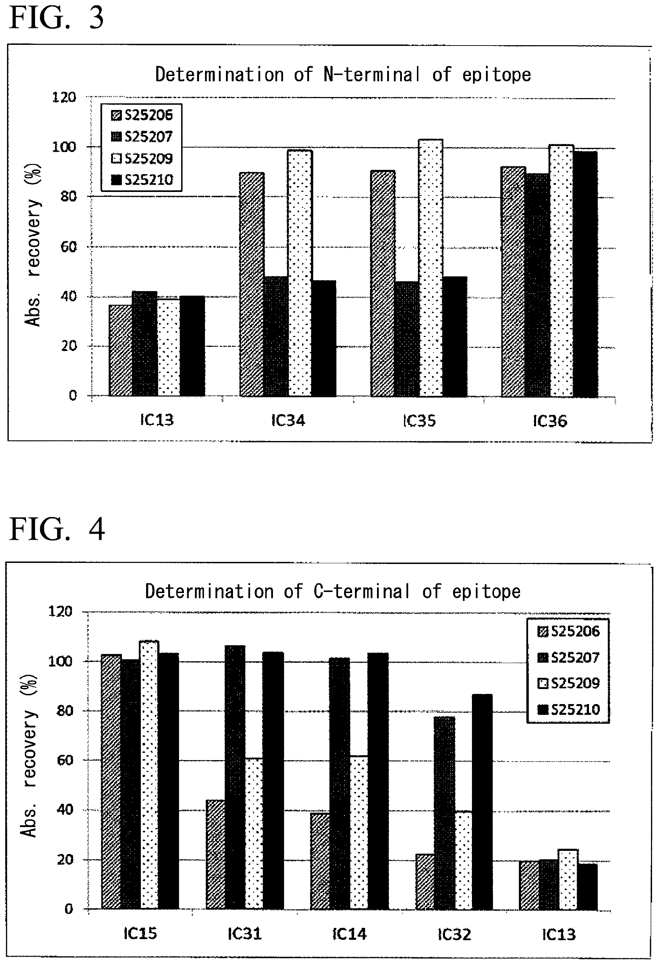

FIG. 3 shows the results of examining the N-terminal side of the amino acid sequence recognized as an epitope by the acquired antibody using the peptides. The results revealed that the S25206 antibody and the S25209 antibody showed reactivity with the peptide IC13, but showed low reactivity with the peptides IC34, IC35, and IC36. Therefore, the N-terminal side of the epitope for these antibodies was considered to be S.

The S25207 antibody and the S25210 antibody showed reactivity with the peptides IC13, IC34, and IC35, but showed low reactivity with the peptide IC36. Therefore, the N-terminal side of the epitope for these antibodies was considered to be G.

Similarly, the results of examining the C-terminal side of the amino acid sequence recognized as an epitope by the acquired antibody are shown in FIG. 4. The results revealed that the S25206 antibody and S25209 antibody showed reactivity with the peptides IC31, IC14, IC32, and IC13, but showed low reactivity with the peptide IC15. Therefore, the C-terminal side of the epitope for these antibodies was considered to be S.

The S25207 antibody and the S25210 antibody showed reactivity with the peptide IC13, but showed low reactivity with the peptides IC15, IC31, IC14, and IC32. Therefore, the C-terminal side of the epitope for these antibodies was considered to be P.

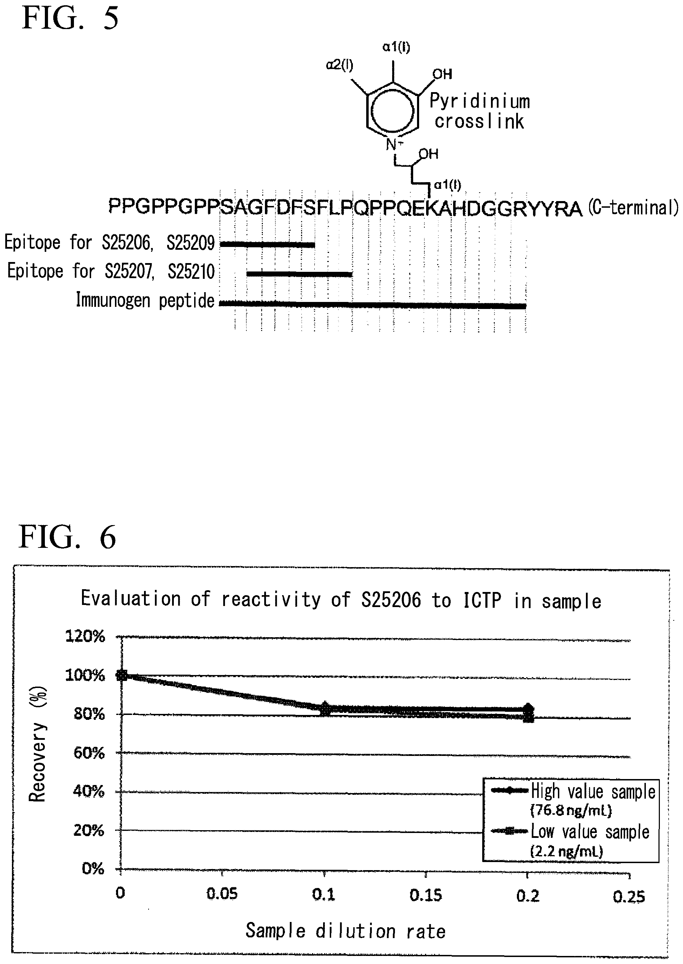

From the above, the epitope for the S25206 antibody and S25209 antibody was considered to be SAGFDFS (SEQ ID NO: 4), and the epitope for the S25207 antibody and S25210 antibody was considered to be GFDFSFLP (SEQ ID NO: 1) (Fig. 5).

### <<Example 2 Evaluation of reactivity with actual samples by competitive ELISA>>

Of the two types of monoclonal antibodies obtained in Example 1 that recognize different epitopes, the S25206 antibody and the S25207 antibody were used in the experiment.

### 2-1 Operation for competitive ELISA

(1) IC01-(PEG)2-BSA (100 ng/mL in PBS) was dispensed onto a plate (50 µL/well) and allowed to stand at room temperature for 2 hours.
(2) After washing 3 times (400 µL/well), a blocking solution was dispensed (100 µL/well) and the resulting was allowed to stand at room temperature for 1 hour.
(3) After removing the blocking solution, the cancer bone metastasis patient serum (5, 10-fold diluted, blank) was dispensed (25 µL/well), followed by further dispensing a purified antibody prepared in-house (10 ng/mL in 1% BSA/PBST) (25 µL/well), and the resulting was allowed to stand at room temperature for 1 hour.
(4) After washing three times, Goat anti-Mouse IgG (H+L) PAb-HRP (9500-fold diluted) was dispensed (50 µL/well) and the resulting was allowed to stand at room temperature for 1 hour.
(5) After washing 3 times, an OPD coloring solution was dispensed (50 µL/well) and the resulting was allowed to stand at room temperature for 10 minutes.
(6) A stop solution was dispensed (50 µL/well), and after stopping the reaction, measurement was performed using a plate reader (Abs. 492 nm).

### 2-2 Results of competitive ELISA

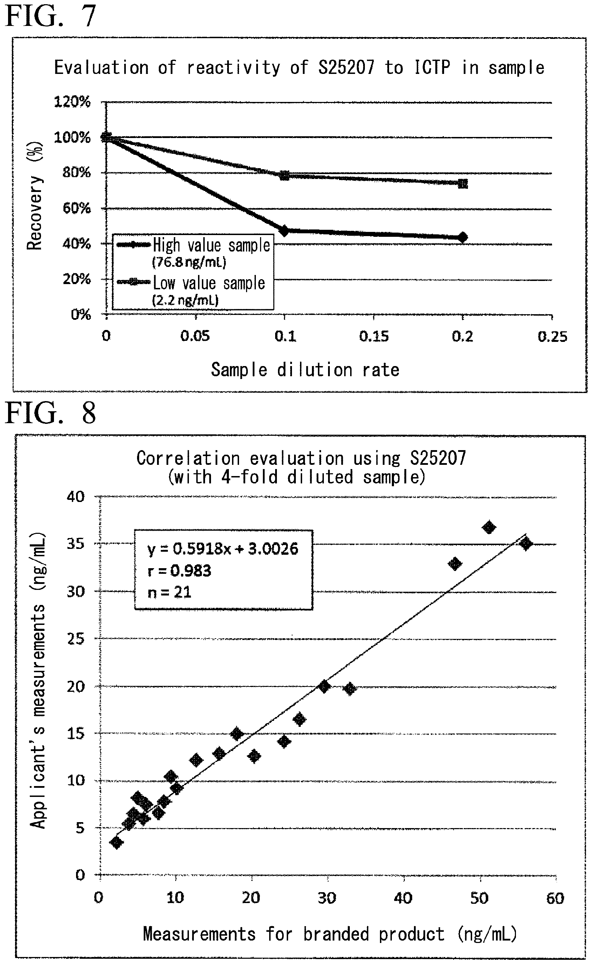

Regarding the S25206 antibody and the S25207 antibody, specimens with low ICTP measurement value (2.2 ng/mL) and specimens with high measurement value (76.8 ng/mL) were used to observe whether the antibody reacts concentration-dependently with ICTP in the specimen. The results for the S25206 antibody are shown in FIG. 6. The results for the S25207 antibody are shown in FIG. 7.

As a result, the S25207 antibody was confirmed to show concentration-dependent reactivity with respect to the high-value specimen and the low-value specimen. For the S25206 antibody, concentration-dependent reactivity was not observed with respect to the high-value specimen and the low-value specimen. Thus, it has been shown that the S25207 antibody binds to ICTP in cancer bone metastasis patient serum.

### <<Example 3 Correlation evaluation by competitive ELISA using S25207 antibody as promising antibody>>

Regarding the S25207 antibody, competitive ELISA measurements were implemented with respect to 21 specimens, and the correlation with a prior commercial product was investigated.

### 3-1 Operation for competitive ELISA

(1) IC01-(PEG)2-BSA (100 ng/mL in PBS) was dispensed onto a plate (50 µL/well) and allowed to stand at room temperature for 2 hours.
(2) After washing 3 times (400 µL/well), a blocking solution was dispensed (100 µL/well) and the resulting was allowed to stand at room temperature for 1 hour.
(3) After removing the blocking solution, a standard prior commercial product (4-fold diluted, blank) and a cancer bone metastasis patient serum (4-fold diluted, blank) were dispensed (25 µL/well). Further, 8 ng/mL of biotinylated S25207 antibody was dispensed (25 µL/well) onto the plate and the resulting was allowed to stand at room temperature for 1 hour (0.1 mg/mL of mouse IgG was used as a non-specific inhibitor).
(4) After washing 3 times (400 µL/well), 0.2 µg/mL of HRP-Streptavidin was dispensed (50 µL/well) and the resulting was allowed to stand at room temperature for 30 minutes.
(5) After washing 3 times (400 µL/well), an OPD coloring solution was dispensed (50 µL/well) and the resulting was allowed to stand at room temperature for 10 minutes.
(6) A stop solution was dispensed (50 µL/well), and after stopping the reaction, measurement was performed using a plate reader with respect to Abs. 492 nm.

### 3-2 Results of competitive ELISA

A calibration curve was created from the measured values of the standard prior commercial product, and the in-house measured ICTP value of the specimen was calculated based on the obtained regression formula. As a result, a good correlation with the pyridinoline ICTP reagent as a prior commercial product was obtained (correlation coefficient r = 0.983) (FIG. 8).

### [Industrial Applicability]

The present invention can provide an immunological assay method using a monoclonal antibody and a kit including the monoclonal antibody, which can be used for assaying ICTP without requiring special facilities.

### [Accession number]

### [Reference to deposited biological material]

(1) Hybridoma S25207 producing antibody number S25207
   (a) Name and address of the depositary with which the biological material was deposited: National Institute of Technology and Evaluation
      2-5-8 Kazusakamatari, Kisarazu, Chiba, Japan (zip/postal code 292-0818)
   (b) Date of deposit of biological material with the depository of (a)
      March 11, 2021
   (c) Accession number assigned to deposit by the depositary of (a):
      NITE BP-03431

## Claims

1. A method for immunological assay of type I collagen C-terminal telopeptide in a biological sample, comprising:
contacting type I collagen C-terminal telopeptide with a monoclonal antibody that recognizes an amino acid sequence represented by GFDFSFLP (SEQ ID NO: 1) as an epitope.

2. The method according to claim 1, wherein the monoclonal antibody does not recognize an amino acid sequence represented by FDFSFLP (SEQ ID NO: 2) as an epitope.

3. The method according to claim 1 or 2, wherein a labeling substance is directly or indirectly bound to the monoclonal antibody.

4. The method according to claim 3, wherein the labeling substance is a metal complex, an enzyme, an insoluble particle, or a metal colloid.

5. The method according to claim 3 or 4, which further comprises a step of measuring a signal generated by the labeling substance.

6. The method according to any one of claims 1 to 5, wherein the biological sample is blood, plasma, or serum.

7. The method according to any one of claims 1 to 6, wherein the biological sample is a biological sample of a subject having breast cancer, prostate cancer, or lung cancer, which has metastasized to bone.

8. The method according to any one of claims 1 to 7, wherein the immunological assay is electrochemiluminescence immunoassay, latex immunoturbidimetry, immunochromatography, or ELISA.

9. A kit for assaying type I collagen C-terminal telopeptide in a biological sample, comprising:
a monoclonal antibody that recognizes an amino acid sequence represented by GFDFSFLP (SEQ ID NO: 1) as an epitope.

10. The kit according to claim 9, wherein the monoclonal antibody does not recognize an amino acid sequence represented by FDFSFLP (SEQ ID NO: 2) as an epitope.

11. The kit according to claim 9 or 10, wherein the biological sample is blood, plasma or serum.

12. The kit according to any one of claims 9 to 11, wherein the biological sample is a biological sample of a subject having breast cancer, prostate cancer, or lung cancer, which has metastasized to bone.

13. The kit according to any one of claims 9 to 12, which is for use in electrochemiluminescence immunoassay, latex immunoturbidimetry, immunochromatography or ELISA.

14. A monoclonal antibody that recognizes an amino acid sequence represented by GFDFSFLP (SEQ ID NO: 1) as an epitope.

15. The monoclonal antibody according to claim 14, which does not recognize an amino acid sequence represented by FDFSFLP (SEQ ID NO: 2) as an epitope.
